## Europäisches Patentamt

## European Patent Office

## Office européen des brevets

(19)

(11) Veröffentlichungsnummer: **0 190 424**
**B1**

(12) **EUROPÄISCHE PATENTSCHRIFT**

(45) Veröffentlichungstag der Patentschrift:
17.10.90

(51) Int. Cl.⁵: **C07C 67/303**, C07C 69/40

(21) Anmeldenummer: 85115159.7

(22) Anmeldetag: 29.11.85

(54) Verfahren zur Herstellung reiner Dialkylsuccinate.

(30) Priorität: 01.02.85 DE 3503485

(43) Veröffentlichungstag der Anmeldung:
13.08.86 Patentblatt 86/33

(45) Bekanntmachung des Hinweises auf die Patenterteilung:
17.10.90 Patentblatt 90/42

(84) Benannte Vertragsstaaten:
AT BE CH DE FR GB IT LI LU NL SE

(56) Entgegenhaltungen:
US-A- 3 830 830

JOURNAL OF ORGANOMETALLIC CHEMISTRY,
Band 26, 1971, Seiten 389-392, Elsevier Sequoia S.A.,
Lausanne, CH; P. TAYLOR et al.: "The isomerization
and hydrogenation of the maleatefumarate system by
cobalt hydrocarbonyl"

(73) Patentinhaber: Chemie-Linz Gesellschaft m.b.H.,
St.Peter-Strasse 25, A-4021 Linz(AT)

(72) Erfinder: Hornich, Heinrich, Mag. pharm. Dr.,
Boschweg 111, A-4020 Linz(AT)
Erfinder: Schaller, Josef, Kaplanhofstrasse 28,
A-4020 Linz(AT)

(74) Vertreter: Kunz, Ekkehard, Dr., Chemie Holding AG
Patentwesen St. Peter-Strasse 25, A-4021 Linz(AT)

ACTORUM AG

## Beschreibung

Die vorliegende Erfindung betrifft ein Verfahren zur Herstellung reiner Dialkylsuccinate durch katalytische Hydrierung der entsprechenden Dialkylmaleinate bzw. Dialkylfumarate.

Dialkylsuccinate werden in der Praxis bevorzugt durch katalytische Hydrierung von Maleinsäureanhydrid, von Maleinsäure und Fumarsäure bzw. deren Monoalkylestern in Anwesenheit von Alkoholen hergestellt. So wird z.B. in der US-PS 3 830 830 ein Verfahren zur Herstellung von Dialkylsuccinaten beschrieben, bei dem in erster Stufe durch Reaktion von Maleinsäureanhydrid mit einem Alkohol das Monoalkylmaleinat gebildet wird, das in einer zweiten Reaktionsstufe bei 25 bis 150°C, während 5 Minuten bis 5 Stunden, bei einem Wasserstoffdruck von 0,3 bis 400 bar in Anwesenheit eines geeigneten Hydrierkatalysators, wie z.B. Palladium, Platin, Rhodium, Ruthenium, Rhenium, Kupferchromit, Kupfer, Kobalt oder Nickel in alkoholischer Lösung zu Monoalkylsuccinat hydriert wird. In dritter Reaktionsstufe wird sodann mit überschüssigem Alkohol weiter zum Dialkylsuccinat verestert, wobei jedoch bei einer Ausbeute von maximal 93% Dimethylsuccinat (Beispiel 1) 7% Monomethylsuccinat als Verunreinigung zurückbleiben. Die fertige Reaktionslösung, die außerdem noch Wasser und überschüssigen Alkohol enthält, muß zur Gewinnung von reinem Dialkylsuccinat anschließend noch auf destillativem Weg aufgearbeitet werden.

Ein Verfahren zur Herstellung von Diisobutylsuccinat durch Einwirken von Wasserstoff auf Lösungen von Maleinsäure bzw. Fumarsäure in Isobutanol wird in der DE-PS 485 313 beschrieben, wobei in Gegenwart von feinverteiltem Nickel bei 100°C und einem Druck von 100 bar gearbeitet wurde. Auch hier muß dann das überschüssige Isobutanol durch Destillation abgetrennt werden.

In der DE-PS 1 493 202 werden Dialkylsuccinate hergestellt, indem die entsprechenden ungesättigten Dicarbonsäuren bzw. ihre Anhydride oder Monoester in einem Alkohol gelöst werden, die erhaltene Lösung verdampft, mit Wasserstoff vermischt und über einem Nickel-Kieselsäure-Kontakt in der Gasphase bei 200 bis 275°C hydriert werden. Auf diese Weise wird z.B. aus Maleinsäureanhydrid und Methanol in 82%iger Ausbeute Dimethylsuccinat gewonnen.

In J. Organometallic Chem. 26 (1971) S. 389–392 werden Dialkylsuccinate durch Umsetzung von Dialkylmaleinaten und Dialkylfumaraten mit Cobalthydrocarbonyl in Toluol als Lösungsmittel unter CO-Atmosphäre in 45–60%iger Ausbeute erhalten.

Alle diese Verfahren besitzen den Nachteil, daß Alkohole als Lösungsmittel bzw. zur Veresterung bzw. organische Lösungsmittel verwendet werden, die nach der Reaktion in einem zusätzlichen Verfahrensschritt wieder aufgearbeitet werden müssen. Außerdem erfordern die bekannten Verfahren wegen der teilweise hohen Temperaturen und Drucke speziell diesen Anforderungen entsprechend aufwendig konstruierte Apparaturen, bzw. sie sind wegen der mehrstufigen Verfahrensweise sehr zeitintensiv, kostenaufwendig und in ihrer Durchführung entsprechend umständlich. Ein weiterer entscheidender Nachteil ist die nicht vollständige Umsetzung der Ausgangsprodukte zum gewünschten Dialkylester. Dadurch ist die Reaktionsmischung einerseits immer durch Nebenprodukte verunreinigt und muß zur Erzielung des reinen Dialkylsuccinats erst entsprechend gereinigt werden. Zum anderen liegt dadurch, wie bereits erwähnt, die Ausbeute an Dialkylsuccinat entsprechend niedrig und beträgt im besten Fall 93%.

Die Aufgabe der vorliegenden Erfindung bestand darin, Dialkylsuccinate unter Ausschaltung der oben angeführten Nachteile in hoher Ausbeute und Reinheit herzustellen. Diese Aufgabe konnte überraschenderweise dadurch gelöst werden, daß die entsprechenden Dialkylmaleinate oder Dialkylfumarate in flüssigem Aggregatzustand bzw. in geschmolzener Form, ohne Verwendung eines Lösungsmittels, katalytisch hydriert wurden.

Gegenstand der Erfindung ist demnach ein Verfahren zur Herstellung von reinen Dialkylsuccinaten durch katalytische Hydrierung von Derivaten der Maleinsäure bzw. Fumarsäure, das dadurch gekennzeichnet ist, daß man die katalytische Hydrierung an den entsprechenden unverdünnten, in flüssigem Aggregatzustand vorliegenden Dialkylmaleinaten und/oder Dialkylfumaraten bei Temperaturen von 40 bis 190°C, mit der Maßgabe, daß die Reaktionstemperatur oberhalb des Schmelzpunktes des eingesetzten Ausgangsmaterials zu liegen kommt und bei einem Druck von 1 bis 40 bar durchführt.

Das erfindungsgemäße Verfahren zeichnet sich demnach vornehmlich dadurch aus, daß in einstufiger Reaktion ohne Verwendung eines Lösungsmittels bereits die reinen Dialkylsuccinate in sehr hohen Ausbeuten von bis zu 99% erhalten werden. Dadurch entfällt die Aufarbeitung des Reaktionsgemisches, wie dies bei den bekannten Verfahren notwendig ist, und es können sowohl Zeit, Energie- und Arbeitskosten als auch der apparative Aufwand für die Durchführung des Verfahrens beträchtlich reduziert werden. Nach der Abtrennung des Katalysators aus dem Reaktionsprodukt werden ohne weitere Reinigungsschritte qualitativ hochwertige Dialkylsuccinate mit einer Reinheit von 99% erhalten. Aufgrund der einstufigen Reaktion gelingt es auch besonders einfach, das erfindungsgemäße Verfahren kontinuierlich zu führen.

Das erfindungsgemäße Verfahren ist besonders interessant zur Herstellung von Ausgangsprodukten für Weichmacher und Antioxydantien, wobei insbesondere Alkylgruppen mit einer Kettenlänge von $C_1$ bis $C_{12}$, besonders bevorzugt von $C_1$ bis $C_8$ in Frage kommen.

Gemäß einer besonders bevorzugten Arbeitsweise werden die Ausgangssubstanzen vor der Hydrierung auf Temperaturen von 40 bis 130°C, mindestens jedoch auf den Schmelzpunkt des eingesetzten Esters vorgewärmt. Die optimale Reaktionstemperatur stellt man dann bei der anschließenden exothermen Hydrierung besonders energiesparend unter Ausnutzung der freiwerdenden Hydrierwärme ohne weitere Wärmezufuhr von außen ein. Bei entsprechender apparativer Ausstattung, sowie bei kontinuierlicher Fahrweise ist es auch möglich, die zum Vorwärmen benötigte Wärmeenergie durch Ausnutzung der überschüssigen Hydrierwärme bereitzustellen, sodaß die gesamte Hydrierung ohne jede Wärmezufuhr von außen durchgeführt werden.

Die optimale Hydriertemperatur ist vom Schmelzpunkt der eingesetzten Ester und von deren Reaktionsfähigkeit abhängig. Für die Herstellung von $C_1$ bis $C_8$-Succinaten sind Hydriertemperaturen von 100 bis 165°C bevorzugt. Sie liegt z.B. beim Diethylmaleinat bzw. Diethylfumarat bei etwa 160°C, beim Dimethylmaleinat hingegen bei etwa 125°C.

Die Hydrierung zu den Dialkylsuccinaten wird vorteilhafterweise bei Drucken von 1 bis 15 bar, besonders bevorzugt bei 6 bis 9 bar durchgeführt, wodurch einerseits das Reaktionsgleichgewicht ausreichend weit in Richtung hydriertes Produkt verlagert wird, anderseits jedoch die Verwendung besonders aufwendiger und teurer Hochdruckapparaturen vermieden werden kann.

Die Hydrierung wird in Gegenwart von geeigneten Hydrierkatalysatoren durchgeführt, wobei sich der Einsatz von Metallen der 8. Gruppe des Periodensystems besonders bewährt hat. Diese Metalle können dabei beispielsweise in feinverteilter Form auf Aktivkohle oder Metalloxiden wie z.B. $Al_2O_3$ aufgebracht sein. So wird z.B. besonders bevorzugt auf Aktivkohle aufgebrachtes Palladium verwendet. Eine weitere Möglichkeit liegt darin, die Hydrierkatalysatoren in feinverteilter, reiner Form einzusetzen.

Das erfindungsgemäße Verfahren wird anhand der angeführten Beispiele erläutert. Die Produktidentifikation erfolgte in allen Beispielen mittels Elementaranalyse, IR-Spektroskopie und NMR-Spektroskopie. Die Reinheit wurde mittels Gaschromatographie und Berechnung der Flächenprozente ermittelt.

Beispiel 1:

In einem 1 l-Autoklaven mit Rührwerk und Mantelkühler werden 576,52 g Dimethylmaleinat bei Raumtemperatur vorgelegt und 5 g eines Pd/C-Katalysators (K 0225, Heraeus, 10 %ig) zugegeben. Der darüberliegende Gasraum wird mit Stickstoff gespült. Danach wird das Reaktionsgemisch auf 40°C vorgewärmt und unter Rühren Wasserstoff mit einem konstanten Druck von 7 bar aufgedrückt. Die bei der Hydrierung auftretende Reaktionswärme (ca. 28 kcal/Mol) wird mittels Wasserkühlung abgeführt und die Reaktionsmischung bei 125°C thermostatisiert. Die Reaktion ist beendet, wenn nach Abstellen des zugeführten Wasserstoffs über 20 Minuten eine Druckkonstanz zu beobachten ist. Die gesamte Reaktionszeit beträgt 90 Minuten. Das Reaktionsgemisch wird sodann auf Raumtemperatur abgekühlt und der eingesetzte Katalysator auf einer Glassinternutsche abgetrennt. Man erhält 576,8 g Dimethylsuccinat (Ausbeute 99,7 %) mit einer Reinheit von 99 % und einem Schmelzpunkt von 17 - 19°C.

Beispiel 2:

In einen 1 l Autoklaven mit Rührwerk und Mantelkühlung werden 654,4 g Diäthylmaleinat und 34,4 g Diäthylfumarat bei Raumtemperatur eingebracht und 5 g Katalysator (Pd/C, 10 %ig) zugegeben. Während dieses Gemisch auf 60°C erwärmt wird, befreit man den darüberliegenden Gasraum mittels Stickstoff vom Luftsauerstoff. Die weitere Reaktionsführung sowie die Aufarbeitung des Reaktionsgemisches erfolgt wie im Beispiel 1 beschrieben, mit dem Unterschied, daß die Temperatur während der Hydrierung bei 160°C gehalten wird. Die Ausbeute an Diäthylsuccinat (Kp = 97 - 99°C) beträgt 692,1 g (99,3 %), die Produktreinheit liegt bei 99,5% GC-Untersuchung).

Beispiel 3:

In einer wie in Beispiel 1 beschriebenen Apparatur werden 523 g Bis-(2-äthylhexyl)fumarat nach Erwärmung auf 70°C wie im Beispiel 1 angegeben, jedoch bei 162°C, hydriert. Nach Reaktionsende erfolgt die Aufarbeitung ebenfalls analog Beispiel 1. Die Ausbeute an Bis-(2-äthylhexyl)-succinat beträgt 98,6 % der Theorie, die Produktreinheit beträgt 99,2 %.

Beispiel 4:

In einer Apparatur gemäß Beispiel 1 werden 422 g kristallines Bis(dodecyl)maleinat vorgelegt und durch Erwärmen auf 70°C in die Schmelze Nach Zugabe von 5 %igem Pd/C als Katalysator erfolgt die Hydrierung (178°C) und Aufarbeitung (Abtrennung des Katalysators allerdings bei 80°C) wie im Beispiel 1 beschrieben. Die Ausbeute an Bis-(dodecyl)-succinat (Fp = 34 -37°C) ist 415,9 g (98,3 %), die Produktreinheit ist 99,3 %.

Beispiel 5:

Wie in Beispiel 4 beschrieben werden 590 g kristallines Dicyclohexylfumarat vorgelegt, auf 118°C vorgewärmt, bei 190°C hydriert und aufgearbeitet. Man erhält 586,9 g Dicyclohexylsuccinat (Ausbeute 99,0 %) mit einer Reinheit von 99,2 %.

Beispiel 6:

Gemäß Beispiel 4 werden 590 g kristallines Dicyclohexylmaleinat vorgelegt, auf 130°C vorgewärmt, bei 172°C hydriert und aufgearbeitet. Die Ausbeute an Dicyclohexylsuccinat beträgt 482,9 g (99,4 %) mit einer Reinheit von 99,7 %.

Beispiel 7:

576,52 g Dimethylmaleinat werden in einer Apparatur entsprechend Beispiel 1 mit einen $H_2$-Druck

von 3 bar und einer Reaktionstemperatur zwischen 40 - 70°C zu Dimethylsuccinat hydriert. Als Katalysator wird Pt/C (F103R/Fa. Degussa, 10 %ig) eingesetzt. Dabei werden 576,2 g Dimethylsuccinat von 99 % Reinheit, entsprechend einer Ausbeute von 99,6 % erhalten.

Beispiel 8:

In einer Apparatur gemäß Beispiel 1 werden 576,52 g Dimethylmaleinat bei 50 - 90°C, einem Hydrierdruck von 15 und unter Verwendung von Ru/C (H 101B/Fa. Degussa, 5 %ig) als Katalysator hydriert. Dabei werden 576,8 g Dimethylsuccinat mit einer Reinheit von 99,5 %, entsprechend einer Ausbeute von 99,7 % erhalten.

Beispiel 9:

576,52 g Dimethylmaleinat werden gemäß Beispiel 1 bei 13 bar $H_2$-Druck und einer Reaktionstemperatur zwischen 70 - 120°C zu 572,75 g Dimethylsuccinat (Ausbeute 99,0 %) hydriert. Als Katalysator wird Rh/C (3/379/Fa. Degussa, 5 %ig) verwendet. Die Reinheit des entstandenen Dimethylsuccinats liegt bei 99,2 %.

Beispiel 10:

576,52 g Dimethylmaleinat werden gemäß Beispiel 1 mit 5 g Ni-Katalysator 5132 P(Fa. Harshaw) bei 9 bar $H_2$-Druck und einer Reaktionstemperatur von 50 - 100°C hydriert. Dabei werden 575,0 g Dimethylsuccinat (Ausbeute: 99,4 %) einer Reinheit von 99,4 % erhalten.

**Patentansprüche**

1. Verfahren zur Herstellung von reinen Dialkylsuccinaten durch katalytische Hydrierung von Derivaten der Maleinsäure und/oder Fumarsäure, dadurch gekennzeichnet, daß man die katalytische Hydrierung an den entsprechenden unverdünnten, in flüssigem Aggregatzustand vorliegenden Dialkylmaleinaten und/oder Dialkylfumaraten bei Temperaturen von 40 - 190°C, mit der Maßgabe, daß die Reaktionstemperatur oberhalb des Schmelzpunktes des eingesetzten Ausgangsmaterials zu liegen kommt und bei einem Druck von 1 bis 40 bar durchführt.

2. Verfahren gemäß Anspruch 1, dadurch gekennzeichnet, daß die Alkylgruppen in den eingesetzten Dialkylestern eine Kettenlänge von $C_1$ bis $C_{12}$ aufweisen.

3. Verfahren gemäß Anspruch 2, dadurch gekennzeichnet, daß die Alkylgruppen eine Kettenlänge von $C_1$ bis $C_8$ aufweisen.

4. Verfahren nach den Ansprüchen 1 bis 3, dadurch gekennzeichnet, daß man das Hydriergemisch vor der Wasserstoffzugabe auf Temperaturen von 40 - 130°C, mindestens jedoch auf den Schmelzpunkt des eingesetzten Esters vorwärmt, und die Reaktionstemperatur unter Ausnützung der bei der Hydrierung auftretenden Wärmetönung ohne weitere Wärmezufuhr einstellt.

5. Verfahren nach den Ansprüchen 3 und 4, dadurch gekennzeichnet, daß die Hydrierung bei einer Temperatur von 100 bis 165°C durchgeführt wird.

6. Verfahren nach den Ansprüchen 1 bis 5, dadurch gekennzeichnet, daß die Hydrierung bei einem Druck von 1 bis 15 bar durchgeführt wird.

7. Verfahren gemäß Anspruch 6, dadurch gekennzeichnet, daß die Hydrierung bei einem Druck von 6 bis 9 bar durchgeführt wird.

8. Verfahren nach den Ansprüchen 1 bis 7, dadurch gekennzeichnet, daß als Hydrierkatalysator ein Metall der 8. Gruppe des Periodensystems eingesetzt wird.

9. Verfahren nach den Ansprüchen 1 bis 7, dadurch gekennzeichnet, daß als Hydrierkatalysator Palladium auf Aktivkohle eingesetzt wird.

**Claims**

1. Process for the preparation of pure dialkyl succinates by catalytic hydrogenation of derivatives of maleic acid and/or fumaric acid, characterized in that the catalytic hydrogenation is carried out on the corresponding undiluted dialkyl maleates and/or dialkyl fumarates, which are in the liquid state of aggregation, at temperatures of 40 to 190°C, with the proviso that the reaction temperature stabilizes above the melting point of the starting material which is used, and under a pressure of 1 to 40 bar.

2. Process according to Claim 1, characterized in that the alkyl groups in the dialkyl esters used have a chain length from $C_1$ to $C_{12}$.

3. Process according to Claim 2, characterized in that the alkyl groups have a chain length from $C_1$ to $C_8$.

4. Process according to Claims 1 to 3, characterized in that before the addition of hydrogen, the hydrogenation mixture is preheated to temperatures of 40 - 130°C, but at least to the melting point of the ester used, and the reaction temperature is set by utilization of the heat evolved during the hydrogenation, without further input of heat.

5. Process according to Claims 3 and 4, characterized in that the hydrogenation is carried out at a temperature of 100 to 165°C.

6. Process according to one of Claims 1 to 5, characterized in that the hydrogenation is carried out under a pressure of 1 to 15 bar.

7. Process according to Claim 6, characterized in that the hydrogenation is carried out under a pressure of 6 to 9 bar.

8. Process according to Claims 1 to 7, characterized in that a metal of the 8th group of the periodic table is used as the hydrogenation catalyst.

9. Process according to Claims 1 to 7, characterized in that palladium on active charcoal is used as the hydrogenation catalyst.

## Revendications

1. Procédé de préparation de dialkylsuccinates purs par hydratation catalytique des dérivés de l'acide maléique et/ou fumarique, caractérisé en ce qu'on réalise l'hydration catalytique des dialkylmaléinates et/ou dialkylfumarates corespondants non dilués, sous forme d'aggrégat liquide, à une température comprise entre 40 et 190°C et à une pression comprise entre 1 et 40 bar, sous réserve que la température de réaction à adopter dépasse le point de fusion des produits de départ utilisés.

2. Procédé selon la revendication 1, caractérisé en ce que les groupes alkyles des dialkylesters utilisés ont une longueur de chaîne de $C_1$ à $C_{12}$.

3. Procédé selon la revendication 2, caractérisé en ce que les groupes alkyles ont une longueur de chaîne de $C_1$ à $C_8$.

4. Procédé selon la revendications 1 à 3, caractérisé en ce qu'on préchauffe le mélange à hydrater, avant introduction d'hydrogène, à une température de 40 à 130°C, au moins cependant au niveau du point de fusion de l'ester utilisé, et en ce qu'on ajuste la température de la réaction en utilisant la formation de chaleur engendrée par l'hydratation, sans apport de chaleur supplémentaire.

5. Procédé selon la revendications 3 et 4, caractérisé en ce que l'hydratation est réalisée à une température comprise entre 100 et 165°C.

6. Procédé selon la revendications 1 à 5, caractérisé en ce que l'hydratation est réalisée à une pression comprise entre 1 et 15 bar.

7. Procédé selon la revendication 6, caractérisé en ce que l'hydratation est réalisée à une pression de 6 à 9 bar.

8. Procédé selon la revendications 1 à 7, caractérisé en ce qu'on utilise un métal du huitième groupe du système périodique comme catalyseur d'hydratation.

9. Procédé selon la revendications 1 à 7, caractérisé en ce qu'on utilise du palladium sur du charbon actif comme catalyseur d'hydratation.